# NEUE EUROPÄISCHE PATENTSCHRIFT

(11) **EP 0 936 913 B2**
(45) Veröffentlichungstag und Bekanntmachung des Hinweises auf die Entscheidung über den Einspruch: **19.09.2012**
(45) Hinweis auf die Patenterteilung: 13.08.2008
(21) Anmeldenummer: 97918146.8
(22) Anmeldetag: 21.04.1997
(51) Int. Cl.: A61K 31/66, A61K 9/20, A61K 9/28

(54) **ORALE PHARMAZEUTISCHE ZUBEREITUNG ENTHALTEND IBANDRONAT**
ORAL PHARMACEUTICAL PREPARATION CONTAINING IBANDRONAT
PREPARATION PHARMACEUTIQUE ORALE CONTENANT DE L'IBANDRONATE

(30) Priorität: 20.04.1996 DE 19615812
(43) Veröffentlichungstag der Anmeldung: 25.08.1999
(60) Teilanmeldung: 06001911.4
(73) Patentinhaber: Roche Diagnostics GmbH, 68305 Mannheim (DE)
(72) Erfinder: MÖCKEL, Jörn, D-69118 Heidelberg (DE); GABEL, Rolf-Dieter, D-68723 Schwetzingen (DE); WOOG, Heinrich, D-69514 Laudenbach (DE)
(74) Vertreter: Vossius & Partner
(86) Internationale Anmeldenummer: PCT/EP1997/001940
(87) Internationale Veröffentlichungsnummer: WO 1997/039755

(56) Entgegenhaltungen:
- EP-A- 0 550 385
- EP-A- 0 566 535
- WO-A-94/12200
- WO-A-94/26310
- WO-A-95/08331
- DE-A- 3 623 397

## Beschreibung

Die Erfindung betrifft pharmazeutische Zubereitungen von Ibandronat oder dessen physiologisch verträglichen Salze zur oralen Applikation, gemäß Anspruch 1.

Der Wirkstoff Ibandronsäure (1-Hydroxy-3-(N-methyl-N-pentyl)aminopropyl-1,1-diphosphonsäure) bzw. deren Salze (Ibandronate) gehören zur Klasse der Diphosphonsäuren, die vor allem von Interesse sind bei der Behandlung von Knochenerkrankungen und bestimmten Störungen des Calciumhaushalts, wie z.B. der Hyperkalzämie, Osteoporose, Tumorosteolyse oder Morbus Paget. Da zur Behandlung der erwähnten Erkrankungen diese Wirkstoffe häufig und über einen langen Zeitraum gegeben werden müssen, ist neben der intravenösen vor allem die orale Applikation anzustreben, da diese bei vielen Patienten auf eine höhere Akzeptanz trifft.

Die orale Behandlung wird jedoch grundsätzlich durch bekannte orale Verträglichkeitsprobleme der Diphosphonsäuren im allgemeinen erschwert. Es ist bekannt, daß Diphosphonsäuren bzw. deren physiologisch unbedenkliche Salze, und insbesondere Aminodiphosphonsäuren, zu Irritationen des oberen Gastrointestinaltrakts führen (Fleisch H, Bisphosphonates in Bone Disease, Herbert Fleisch, Bern 1993; S. 126 - 131). Dies trifft auch zu für Diphosphonate, die auch in relativ geringen Dosierungen von beispielsweise weniger als 50 mg pro Einzeldarreichungsform eingenommen werden. In WO 93/09785 wird darauf hingewiesen, daß beispielsweise der Wirkstoff Risedronat ([1-Hydroxy-2-(3-pyridinyl)-ethyliden]bis-phosphonat) zu Erosionen und Ulzerationen in den oberen Abschnitten des Verdauungstrakts führen kann. Verschiedene Literaturstellen weisen ferner auf gastrointestinale Unverträglichkeiten des Wirkstoffes Pamidronat (Dodwell D et al., Biochemical Effects, Antitumor Activity and Pharmacokinetics of Oral and Intravenous Pamidronate (APD) in the Treatment of Skeletal Breast Cancer, Br. J. Cancer 62, 496 (1990)) und Tiludronat (Reginster J Y, Efficacy and Tolerability of a New Formulation of Oral Tiludronate (Tablet) in the Treatment of Paget's Disease of Bone, J. Bone Miner. Res. 9, 615 - 619 (1994)) hin. Es ist auch bekannt, daß außerdem Motalitätsstörungen beim Schlucken der Tabletten auftreten können bzw. die einzunehmenden Tabletten wegen besonderer anatomischer Gegenheiten im Ösophagus (Speiseröhre) hängen bleiben. Dabei können eine Odynophagie oder auch ösophageale Strikturen entstehen. Dies ist häufig der Fall bei älteren Patienten oder bei Patienten, die infolge ihrer Erkrankung die erforderlichen Tabletten vorwiegend im Liegen einnehmen müssen.

Dementsprechend wurde zur Lösung dieser Probleme in der Fachwelt die Forderung aufgestellt, oral verfügbare Darreichungsformen grundsätzlich mit einem magensaftresistenten Film zu überziehen, so daß der Wirkstoff erst nach der Passage durch den Magen freigesetzt wird, und somit Reizungen des Magens und der Speiseröhre vermieden werden. Beispielsweise werden in WO 95/08331 Darreichungsformen beschrieben, mit denen das Irritationspotential von Alendronat und anderen Diphosphonaten bei oraler Applikation verringert werden kann.

Aufgrund der beschriebenen oralen Unverträglichkeiten der Diphosphonate wurde bei einer Reihe dieser Wirkstoffe nach besser verträglichen Darreichungsformen gesucht. Dabei wurden insbesondere solche oralen Darreichungsformen entwickelt, die mit magensaftresistenten Überzügen überzogen sind. Derartige Überzüge sind das Mittel der Wahl um die oberen Abschnitte des Gastrointestinaltrakts, insbesondere die Speiseröhre oder den Magen, vor unverträglichen Wirkstoffen zu schützen. Diese magensaftresistenten Überzüge auf festen oralen Darreichungsformen lösen sich erst bei einem höheren pH-Wert ab ca. 5,5 auf, so daß im sauren Milieu des Magens, bei einem pH-Wert weit unter 5,5 kein Wirkstoff aus der Darreichungsform abgegeben wird und somit der Magen vor Irritationen durch den Wirkstoff geschützt ist. Da kein Wirkstoff im Magen freigesetzt wird, kann gleichzeitig verhindert werden, daß es durch Reflux wirkstoffhaltigen Mageninhalts zu Ösophagitiden oder anderen Irritationen der Speiseröhre kommt. Folgerichtig wird in DE 59 005 517 (EP 0 421 921) eine magensaftresistente orale Darreichungsform für Pamidronat beschrieben, die geeignet ist, das Risiko von Magenulzera zu reduzieren. WO 93/09785 schildert ferner eine solche Darreichungsform für Risedronat, WO 95/08331 für Alendronat und andere Diphosphonate.

Den möglichen Vorteilen der magensaftresistenten Darreichungsformen stehen eine Reihe von Nachteilen gegenüber. So kann bei diesen Arzneiformen die Resorption gegenüber den pH-unabhängig schnell freisetzenden Formen verringert sein oder die Resorption ist im Vergleich zu konventionellen Formen von wesentlich höherer Variabilität und damit die Therapiesicherheit beeinträchtigt oder in Frage gestellt. Daher besteht Bedarf an einer alternativen Darreichungsform für derartige Wirkstoffe, um die Nachteile der magensaftresistenten Formen zu umgehen, aber dennoch ausreichenden Schutz vor diesen auf die Magenschleimhaut aggressiv wirkenden Aminobisphosphonaten bieten zu können.

Überraschenderweise wurde nun gefunden, daß im Falle des Wirkstoffes Ibandronat bereits dann eine verbesserte orale Verträglichkeit erzielt wird, wenn perorale Darreichungsformen derart mit einer Hilfsstoffschicht oder einem Film überzogen werden, daß sich der Wirkstoff in kurzer Zeit auflöst und entsprechend hohe lokale Konzentrationen des Wirkstoffes im Magen erzielt werden. Der sich beim Kontakt mit den Verdauungssäften auflösende Film ist vorzugsweise ein Überzug, der sich unabhängig vom pH-Wert auflöst. Das Aufbringen dieser Hilfsstoffschicht kann mit pharmazeutisch-technologisch üblichen Verfahren erfolgen. Obwohl diese Schicht nicht die Auflösung des Ibandronats im Magen verhindert, wurden in klinischen Studien überraschenderweise auch mit hochdosiertem Ibandronat keine signifikanten Nebenwirkungen beobachtet. Trotz der Tatsache, daß die Darreichungsformen mit einem den Wirkstoff schnell freisetzenden Film überzogen sind, kommt es beim Schlucken der Tabletten nicht zu Reizungen in der Speiseröhre und es treten keine Ösophagitiden auf. Dies ist insbesondere von Vorteil, da die erfindungsgemäßen Darreichungsformen auch von im Bett liegenden Patienten gut vertragen werden.

Beschrieben werden Filmtabletten von Ibandronat, die aus einem wirkstoffhaltigen Kern bestehen, der mit einer wirkstoff-freien Hilfsstoffschicht überzogen ist, die bei Kontakt mit Verdauungssäften entweder unabhängig vom pH-Wert in Lösung geht oder sich von der festen oralen Zubereitung ablöst. Hierdurch wird sichergestellt, daß die Arzneiform relativ schnell zerfällt und der Wirkstoff in kurzer Zeit freigesetzt wird, wodurch hohe lokale Konzentrationen des Wirkstoffes erzielt werden. Der Überzug kann durch Verfahren wie Filmcoating aufgebracht werden. Die Freisetzung des Wirkstoffs aus den entsprechend überzogenen festen Filmtabletten erfolgt im Vergleich zu den magensaftresistenten Darreichungsformen beschleunigt. Erfindungsgemäß werden mindestens 30 % der enthaltenen Ibandronat-Dosis, bevorzugt aber mindestens 75 % und insbesondere mindestens etwa 85 % innerhalb der physiologischen pH-Bandbreite unabhängig vom pH-Wert schnell freigesetzt. Die Zeit, innerhalb der diese Prozentsätze in der Freisetzung erreicht werden, beträgt vorzugsweise weniger als etwa 2 Stunden, vorzugsweise weniger als 1 Stunde, besonders bevorzugt etwa 1 - 30 Minuten. Besonders bevorzugt beträgt die Freisetzung etwa 80 - 90 % innerhalb einer Zeit von bis zu 15 Minuten. Die Freisetzung des Wirkstoffes wird zweckmäßigerweise im Rahmen eines in vitro Versuches nach allgemein bekannten standardisierten Verfahren bestimmt. Beansprucht werden die Filmtabletten gemäß Beispiel 1.

Die schnelle Freisetzung des Wirkstoffes führt überraschenderweise trotz der dadurch entstehenden hohen lokalen Konzentration des Wirkstoffes (d.h. trotz hohem Wirkstoffgradienten) im Magen nicht zu den üblicherweise für Disphosphonsäure bekannten unerwünschten Nebenwirkungen wie oben beschrieben. Vielmehr wurde gefunden, daß trotz der raschen Freisetzung des Wirkstoffes das Problem des Auftretens gastrointestinaler Störungen völlig überraschend vermieden wird. Außerdem wurde beobachtet, daß bei den mit derartigen schnell zersetzenden Darreichungsformen von Ibandronat behandelten Patienten deutlich weniger Fälle an Übelkeit, Erbrechen, Schmerzen oder Diarrhöe zu beobachten waren, die sonst bei der Verabreichung von Aminobisphosphonaten beobachtet wurden.

Die im Sinne der vorliegenden Erfindung den Wirkstoff schnell freisetzenden Überzüge können auf Tabletten, aufgetragen werden. Diese Filmtabletten bestehen aus Mischungen von Wirkstoffen mit pharmazeutischen Hilfsstoffen oder aus reinen Wirkstoffen. Der Überzug kann mit verschiedenen pharmazeutisch üblichen Verfahren erfolgen. Geeignete Verfahren nutzen Anlagen für das Filmcoating,

Nachfolgend soll die Erfindung durch Ausführungsbeispiele verdeutlicht werden.

Der Wirkstoff Ibandronat wird in Mengen von 10,0 mg, 20,0 mg oder 50,0 mg pro Einzeldosierungseinheit eingesetzt.

### Beispiel 1

| | | | |
|---|---|---|---|
| Ibandronathaltiger Kern: Ibandronat-Dosis [mg] in 200 mg-Tablettenkern: | 10,0 | 20,0 | 50,0 |
| Ibandronatfreie Hülle [mg]: Methylhydroxypropylcellulose | 5,1425 | 5,1425 | 5,1425 |
| Titandioxid | 2,4650 | 2,4650 | 2,4650 |
| Macrogol | 1,5000 | 1,5000 | 1,5000 |
| Talkum | 0,8925 | 0,8925 | 0,8925 |
| Filmauftrag gesamt | 10,0000 | 10,0000 | 10,0000 |

| | | | |
|---|---|---|---|
| Das Filmcoating erfolgt in üblichen Apparaten. Coatingbedingungen: Tabletten-Einlage: 140 kg; Zulufttemperatur: 60 °C. | | | |

### Beispiel 2: (Vergleichsbeispiele)

| | | | |
|---|---|---|---|
| Ibandronathaltiger Kern: Ibandronat-Dosis in 100 mg-Tablettenkern [mg]: | 0,1 | 2,5 | 5,0 |
| Ibandronatfreie Hülle [mg]: Methylhydroxypropylcelulose | 2,057 | 2,057 | 2,057 |
| Titandioxid | 0,986 | 0,986 | 0,986 |
| Macrogol | 0,600 | 0,600 | 0,600 |
| Talkum | 0,357 | 0,357 | 0,357 |
| Filmauftrag gesamt | 4,000 | 4,000 | 4,000 |

| | | | |
|---|---|---|---|
| Filmcoating in konventionellem 250 1-Dragierkessel. Coatingbedingungen: Tabletten-Einlage 144 kg. | | | |

### Beispiel 3: (Vergleichsbeispiel)

| | | | |
|---|---|---|---|
| Ibandronathaltiger Kern: Ibandronat-Dosis [mg] in 200 mg-Tablettenkern: | 10,0 | 20,0 | 50,0 |
| Ibandronatfreie Hülle [mg]: | | | |
| Talkum | 2,00 | 2,00 | 2,00 |
| Lactose | 1,40 | 1,40 | 1,40 |
| Methylhydroxypropylcellulose | 0,80 | 0,80 | 0,80 |
| Titandioxid | 0,80 | 0,80 | 0,80 |
| Macrogol | 0,40 | 0,40 | 0,40 |
| Ethylmethylmethacrylat Copolymer * | 0,04 | 0,04 | 0,04 |
| Polysorbat | 0,04 | 0,04 | 0,04 |
| Filmauftrag gesamt | 5,48 | 5,48 | 5,48 |

| | | | |
|---|---|---|---|
| ***** eingesetzt in Form von Eudragit^{™} NE 30 D als wäßrige Dispersion Filmcoating in konventionellem 15 1-Dragierkessel. Coatingbedingungen: Tabletten-Einlage: 13,0 kg. | | | |

### Bespiel 4: (Vergleichs beisplel)

| | |
|---|---|
| Ibandronathaltiger Kern: Ibandronat-Dosis in 74 mg-Tablettenkern [mg]: | 20 |
| Ibandronatfreie Hülle [mg]: | |
| Methylhydroxypropylcellulosephthalat | 4,580 |
| Triacetin | 1,374 |
| Polysorbat | 0,046 |
| Filmauftrag gesamt | 6,000 |
| Filmcoating in konventionellem 5 1-Dragierkessel. Coatingbedingungen: Tabletten-Einlage: 0,25kg. | |

### Beispiel 5: (Vergleichsbeispiel)

| | |
|---|---|
| Ibandronathaltiger Kern: Ibandronat-Dosis [mg] in 86 mg-Tablettenkern: | 10 |
| Ibandronatfreie Hülle [mg]: | |
| Saccharose | 37,844 |
| Weisser Ton | 8,138 |
| Talkum | 1,000 |
| Macrogol | 2,848 |
| Glucosesirup | 2,035 |
| Titandioxid | 1,628 |
| Polyvidon | 0,407 |
| Montanglycolwachs | 0,100 |
| Dragierauftrag gesamt | 54,000 |

| | |
|---|---|
| Dragierung in konventionellem 15 1-Dragierkessel mit Tauchrohr. | |

### Beispiel 6: (Vergleichsbeispiel)

Ibandronathaltiger Kern:
Ibandronat-Dosis [mg] in 400 mg-Granulat: 20 mg und 50 mg
Ibandronatfreie Hülle [mg]: Hartgelatinesteckkapsel Größe 0, weiß opak.

Die Verkapselung der Arzneimittelmasse erfolgt auf einer Kapselmaschine vom Typ Harro-Höfliger.

### Beispiel 7: (Vergleichsbeispiel)

| | |
|---|---|
| Ibandronat in Manteltablette | |
| | |
| Ibandronathaltiger Kern: | |
| Endgewicht: | 86 mg |
| Kernformat: | 7 mm Durchmesser, plan, facettiert |
| Ibandronat-Dosis in Tablettenkern: | 10 mg |
| | |
| Ibandronatfreie Hülle : | |
| Lactose | 270 mg |
| Mikrokristalline Cellulose: | 90 mg |
| Preßwerkzeug: | 12 mm Durchmesser |
| | |
| Endgewicht Manteltablette: | 446 mg |

| | |
|---|---|
| Das Verpressen erfolgt mitttels Handpresse nach konventionellen Verfahren. | |

## Patentansprüche

1. Filmtablette, bestehend aus einem 200 mg Ibandronat-haltigen Kern mit einer Ibandronat-Dosis von 10,0 mg, 20,0 mg oder 50,0 mg und einem Film, der aus
| | |
|---|---|
| 5.1425 mg | Methylhydroxypropylcellulose, |
| 2.4650 mg | Titandioxid, |
| 1.5000 mg | Macrogol und |
| 0.8925 mg | Talkum besteht. |

2. Verwendung eines Films, der aus 5.1425 mg Methylhydroxypropylcellulose, 2.4650 mg Titandioxid, 1.5000 mg Macrogol und 0.8925 mg Talkum besteht, zur Herstellung einer Filmtablette, die einen 200 mg Ibandronat-haltigen Kern mit einer Ibandronat-Dosis von 10,0 mg, 20,0 mg oder 50,0 mg hat, für die Behandlung von Calciumstoffwechselerkrankungen unter Vermeidung von Ösophagitiden.

## Claims

1. Film tablet consisting of an ibandronate-containing core of 200 mg with an ibandronate dose of 10.0 mg, 20.0 mg or 50 mg and a film consisting of
5.1425 mg methylhydroxypropylcellulose
2.4650 mg of titanium dioxide
1.5000 mg of macrogol, and
0.8925 mg of talc.

2. Use of a film consisting of 5.1425 mg methylhydroxypropylcellulose, 2.4650 mg of titanium dioxide, 1.5000 mg macrogol and 0.8925 mg of talc in the manufacture of a film tablet having an ibandronate-containing core of 200 mg with an ibandronate dose of 10.0 mg, 20.0 mg or 50 mg for treating disorders in calcium metabolism while avoiding oesophagites.

## Revendications

1. Comprimé pelliculé consistant en un noyau de 200 mg contenant de l'ibandronate avec une dose d'ibandronate de 10,0 mg, 20,0 mg ou 50 mg et en une pellicule qui consiste en
5.1425 mg de méthylhydroxypropylcellulose,
2.4650 mg de dioxyde de titane,
1.5000 mg de macrogol, et
0.8925 mg de talc.

2. Utilisation d'une pellicule qui consiste en 5.1425 mg de méthylhydroxypropylcellulose, 2.4650 mg de dioxyde de titane, 1.5000 mg de macrogol, et 0.8925 mg de talc, pour la préparation d'un comprimé pelliculé ayant un noyau de 200 mg contenant de l'ibandronate avec une dose de 10,0 mg, 20,0 mg ou 50 mg, pour le traitement des maladies du métabolisme du calcium en évitant les oesophagites.
